# FASCICULE DE BREVET EUROPEEN

(11) **EP 1 001 789 B1**
(45) Date de publication et mention de la délivrance du brevet: **06.11.2002**
(21) Numéro de dépôt: 98941526.0
(22) Date de dépôt: 03.08.1998
(51) Int. Cl.: A61K 31/70, A61K 31/505, A61P 31/18

(54) **PRODUIT COMPRENANT AU MOINS UN ARN DOUBLE BRIN EN ASSOCIATION AVEC AU MOINS UN DERIVE DU 6-BENZYLURACIL POUR UNE UTILISATION THERAPEUTIQUE SIMULTANEE, SEPAREE OU ETALEE DANS LE TEMPS**
ZUSAMMENSETZUNG DIE MINDESTENS EINEM DOPPEL STRANGIGER RNA MIT MINDESTENS EINEM 6-BENZYLURACILE DERIVAT ENTHÄLT FÜR EINE GLEICHZEITIGE, GETRENNTE ODER ZEITAUSGELEGTE THERAPEUTISCHE VERWENDUNG
PRODUCT COMPRISING AT LEAST A DOUBLE STRANDED RNA COMBINED WITH AT LEAST A 6-BENZYLURACIL DERIVATIVE FOR A SIMULATNEAOUS, SEPARATED OR TIME-SPREADED THERAPEUTIC USE

(30) Priorité: 04.08.1997 FR 9709974
(43) Date de publication de la demande: 24.05.2000
(73) Titulaire: SOCIETE DE CONSEILS DE RECHERCHES ET D'APPLICATIONS SCIENTIFIQUES (S.C.R.A.S.), 75016 Paris (FR)
(72) Inventeur: RUHLMANN, Eric, F-75016 Paris (FR)
(74) Mandataire: Bourgouin, André
(86) Numéro de dépôt international: FR9801726
(87) Numéro de publication internationale: WO99007384

(56) Documents cités:
- EP-A- 0 286 224
- GILLESPIE D ET AL: "An hypothesis concerning optimal therapy in HIV disease." MED HYPOTHESES, JAN 1992, 37 (1) P1-5, XP002089595
- BIOLOGICAL ABSTRACTS, vol. 1995, Philadelphia, PA, US; abstract no. 09776755, BRENNAN TARA M STANLEY: "The inhibition of human immunodeficiency virus type 1 in vitro by a non-nucleoside reverse transcriptase inhibitor MKC-442, alone and in combination with other anti-HIV compounds." XP002089596 & ANTIVIRAL RESEARCH, 1995, vol. 26, no. 2, pages 173-187,
- BIOLOGICAL ABSTRACTS, vol. 1996, Philadelphia, PA, US; abstract no. 10462633, OKAMOTO MIKA SATOSHI: "Complete inhibition of viral breakthrough by combination of MKC-442 with AZT during a long-term culture of HIV-1-infected cells." XP002089597 & ANTIVIRAL RESEARCH, 1996, vol. 31, no. 1-2, pages 69-77,
- BIOLOGICAL ABSTRACTS, vol. 1995, Philadelphia, PA, US; abstract no. 09901309, BABA MASANORI_(A) SATOSHI: "HEPT derivatives: 6-benzyl-1-ethoxymethyl-5-isopropyluracil (MKC-442)." XP002089598 & NUCLEOSIDES & NUCLEOTIDES, 1995, vol. 14, no. 3-5, pages 575-583,

## Description

L'invention concerne un produit comprenant au moins un ARN double brin en association avec au moins un dérivé du 6-benzyluracile pour une utilisation thérapeutique simultanée, séparée ou étalée dans le temps. Ce produit peut éventuellement être associé à un autre agent anti-viral. Ces produits sont particulièrement intéressants pour le traitement du SIDA (Syndrome de l'Immuno Déficience Acquise).

Le brevet français No. 2 622 586 décrit un procédé de préparation d'homopolymères et copolymères de polynucléotides et de leurs complexes. Bien que ces composés ne soient pas nouveaux, les procédés antérieurs n'étaient pas capables de fournir de tels complexes à un coût économiquement acceptable et sans impuretés toxiques, ce qui les rendaient inappropriés pour un usage pharmacologique. Le procédé, décrit dans le brevet français ci-dessus, conduit à des produits d'une pureté suffisante pour un usage pharmacologique et nommément décrits pour le traitement des tumeurs.

Le complexe du Poly(A).Poly(U) (c'est-à-dire l'acide polyadénylique complexé avec l'acide polyuridylique) a été décrit comme un agent anti-viral peu actif sur des souches de virus différentes de celles des rétrovirus responsables du Sida (cf. "Effects of polynucleotides on monkeys and man", CLINICAL ASPECTS OF INTERFERONS, 1988, pages 319-331); par conséquent, il n'était pas évident que le Poly(A).Poly(U) puisse être un agent anti-rétroviral, les virus et rétrovirus étant considérés comme des espèces distinctes.

Il a été prouvé que le complexe Poly(A).Poly(U) est aussi un agent inhibiteur puissant des différents Virus de l'Immuno-déficience Humaine (VIH) dans les cultures cellulaires, en bloquant l'entrée du virus. De préférence, le Poly(A).Poly(U) est préparé selon le procédé décrit dans le brevet français No. 2 622 586.

Il a été aussi prouvé que le complexe Poly(A).Poly(U) est efficace lorsqu'il est administré avec d'autres agents anti-SIDA, tels que la 3'-azido-3'-déoxythymidine (AZT), la Didéoxyinosine (DDI) ou la Didéoxycytidine (DDC), car il accroît de façon synergique l'effet de ces derniers (cf. la demande de brevet européen EP 0 509 906). La demanderesse a entre-temps découvert que l'association de dérivés du 6-benzyluracile (décrits notamment dans les demandes de brevet européen EP 0 420 763 et EP 0 631 783) avec des ARN double brin, avec éventuellement d'autres agents anti-viraux comme l'AZT, la DDI ou la DDC, procure un effet synergique inattendu.

Par dérivé du 6-benzyluracile, on entend notamment tous les dérivés de ce type décrits dans les demandes de brevet européen EP 0 420 763 et EP 0 631 783.

L'invention concerne donc un produit comprenant au moins un ARN double brin en association avec au moins un dérivé du 6-benzyluracile pour une utilisation thérapeutique simultanée, séparée ou étalée dans le temps, associés éventuellement à un autre agent anti-viral, et plus particulièrement un agent anti-SIDA.

De préférence, le ou les dérivés du 6-benzyluracile seront des dérivés du 5-isopropyl-6-benzyluracile. Comme dérivé du 6-benzyluracile pour l'invention, on préférera tout particulièrement le 1-éthoxyméthyl-5-isopropyl-6-benzyluracile.

En ce qui concerne les ARN double brin, on préfère employer le poly(A)-poly(U), mais on peut aussi employer un complexe de l'acide polyinosinique avec l'acide polycytidylique, également connu sous le nom de poly(I)-poly(C), ainsi que ces mêmes complexes modifiés par introduction d'acide uridylique dans la chaîne de l'acide polycytidylique (pour une description de ces produits, se référer notamment à la demande de brevet européen EP 0 300 680).

De préférence, la composition du produit selon l'invention comprend de 90 à 100 % d'ARN double brin, de préférence du complexe poly(A)-poly(U), associé à au moins un dérivé du 6-benzyluracile.

De façon plus préférentielle, ladite composition du produit selon l'invention comprend de 99,00 à 99,99 % d'ARN double brin, de préférence du complexe poly(A)-poly(U), associé à au moins un dérivé du 6-benzyluracile.

De façon optionnelle, le produit selon l'invention peut contenir en outre au moins un autre agent thérapeutique, et plus particulièrement au moins un autre agent anti-viral.

De préférence, ledit agent anti-viral agit sur le virus VIH et est par exemple choisi parmi l'AZT, la DDI ou la DDC.

L'invention concerne encore un produit tel que décrit précédemment, caractérisé en ce qu'il comprend de 99,00 à 99,99 % du complexe Poly(A).Poly(U) et de 1,00 à 0,01 % de dérivé(s) du 6-benzyluracile, associés éventuellement à un ou des autres agents anti-viraux.

L'invention concerne en outre une composition pharmaceutique contenant, à titre de principe actif, un produit selon l'invention associé à des diluants ou excipients pharmacologiquement acceptables.

Les compositions pharmaceutiques selon l'invention peuvent être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques selon l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

Enfin, un autre aspect de l'invention concerne l'utilisation d'un produit selon l'invention, et tel que décrit ci-dessus, produit comprenant au moins un ARN double brin, et au moins un dérivé du 6-benzyluracile, pour fabriquer un médicament destiné à traiter le SIDA. De préférence, le produit utilisé comprend du 1-éthoxyméthyl-5-isopropyl-6-benzyluracile.

Le mode d'administration d'un produit selon l'invention est choisi parmi les modes d'administration classiques. Ainsi, l'administration d'ARNdb peut par exemple se faire par voie topique, orale, parentérale, par injection intramusculaire ou intraveineuse. De même, l'administration des dérivés du 6-benzyluracile ou des autres agents anti-viraux peut se faire selon les mêmes voies. Pour chacun de ces composés, l'homme du métier choisira la méthode d'administration la plus appropriée.

La dose d'administration envisagée pour le médicament selon l'invention est comprise entre 0,1 mg à 10 g suivant le type de composé actif utilisé.

A moins qu'ils ne soient définis d'une autre manière, tous les termes techniques et scientifiques utilisés ici ont la même signification que celle couramment comprise par un spécialiste ordinaire du domaine auquel appartient cette invention.

## Revendications

1. Produit comprenant au moins un ARN double brin en association synergique avec au moins un dérivé du 6-benzyluracile pour une utilisation thérapeutique simultanée, séparée ou étalée dans le temps.

2. Produit selon la revendication 1, **caractérisé en ce que** l'ARN double brin est le poly(A)-poly(U).

3. Produit selon la revendication 1, **caractérisé en ce que** l'ARN double brin est le poly(I)-poly(C).

4. Produit selon l'une des revendications 1 à 3, **caractérisé en ce qu'**il comprend en outre au moins un autre agent anti-viral.

5. Produit selon la revendication 4, **caractérisé en** qu'il comprend au moins un agent anti-viral choisi parmi l'AZT, la DDI ou la DDC.

6. Produit selon l'une des revendications 1 à 5, **caractérisé en ce qu'**il comprend du 1-éthoxyméthyl-5-isopropyl-6-benzyluracile.

7. Produit selon l'une quelconque des revendications 1 à 6, **caractérisé en ce qu'**il comprend de 90 à 100 % d'ARN double brin, de préférence du complexe poly(A)-poly(U), associé à au moins un dérivé du 6-benzyluracile.

8. Produit selon l'une quelconque des revendications 1 à 7, **caractérisé en ce qu'**il comprend de 99,00 à 99,99 % du complexe Poly(A).Poly(U) et de 1,00 à 0,01 % de dérivé(s) du 6-benzyluracile, associés éventuellement à un ou des autres agents anti-viraux.

9. Composition pharmaceutique contenant, à titre de principe actif, un produit selon l'une quelconque des revendications 1 à 8 associé à des diluants ou excipients pharmacologiquement acceptables.

10. Utilisation d'un produit selon l'une quelconque des revendications 1 à 8, pour fabriquer un médicament destiné à traiter le SIDA.

11. Utilisation selon la revendication 10, **caractérisée en ce que** le produit utilisé comprend du 1-éthoxyméthyl-5-isopropyl-6-benzyluracile.

## Claims

1. Product containing at least one double-stranded RNA in synergic combination with at least one 6-benzyluracil derivative for therapeutic use, simultaneously, separately or over a period of time.

2. Product according to claim 1, **characterized in that** the double-stranded RNA is poly(A)-poly(U).

3. Product according to claim 1, **characterized in that** the double-stranded RNA is poly(I)-poly(C).

4. Product according to one of claims 1 to 3, **characterized in that** it contains in addition another antiviral agent.

5. Product according to claim 4, **characterized in that** it contains at least one antiviral agent chosen from AZT, DDI or DDC.

6. Product according to one of claims 1 to 5, **characterized in that** it contains 1-ethoxymethyl-5-isopropyl-6-benzyluracil.

7. Product according to any one of claims 1 to 6, **characterized in that** it contains from 90 to 100 % of double-stranded RNA, preferably poly(A)-poly(U) complex, combined with at least one 6-benzyluracil derivative.

8. Product according to any one of claims 1 to 7, **characterized in that** it contains from 99.00 to 99.99 % of poly(A)-poly(U) complex and from 1.00 to 0.01 % of 6-benzyluracil derivative(s), optionally combined with one or more antiviral agents.

9. Pharmaceutical composition containing as active ingredient a product according to any one of claims 1 to 8, combined with pharmacologically acceptable diluents or excipients.

10. Use of a product according to any one of claims 1 to 8 to manufacture a medicament intended for the treatment of AIDS.

11. Use according to claim 10, **characterized in** the product used contains 1-ethoxymethyl-5-isopropyl-6-benzyluracil.

## Patentansprüche

1. Produkt, umfassend mindestens eine zweisträngige RNA in synergetischer Kombination mit mindestens einem Derivat von 6-Benzyluracil für eine gleichzeitige, getrennte oder zeitlich verteilte therapeutische Verwendung.

2. Produkt nach Anspruch 1, **dadurch gekennzeichnet, dass** die zweisträngige RNA Poly(A)-Poly(U) ist.

3. Produkt nach Anspruch 1, **dadurch gekennzeichnet, dass** die zweisträngige RNA Poly(I)-Poly(C) ist.

4. Produkt nach einem der Ansprüche 1 bis 3, **dadurch gekennzeichnet, dass** es außerdem mindestens ein anderes antivirales Mittel umfasst.

5. Produkt nach Anspruch 4, **dadurch gekennzeichnet, dass** es mindestens ein anderes antivirales Mittel umfasst, das aus AZT, DDI oder DDC ausgewählt ist.

6. Produkt nach einem der Ansprüche 1 bis 5, **dadurch gekennzeichnet, dass** es l-Ethoxymethyl-5-isopropyl-6-benzyluracil umfasst.

7. Produkt nach einem der Ansprüche 1 bis 6, **dadurch gekennzeichnet, dass** es 90 bis 100% zweisträngige RNA, vorzugsweise Komplex Poly(A)-Poly(U), umfasst, mit der mindestens ein 6-Benzyluracil-Derivat kombiniert ist.

8. Produkt nach einem der Ansprüche 1 bis 7, **dadurch gekennzeichnet, dass** es 99,00 bis 99,99% des Komplexes Poly(A)-Poly(U) und 1,00 bis 0,01% 6-Benzyluracil-Derivat(e) umfasst, mit denen ggf. ein oder andere antivirale Mittel kombiniert sind.

9. Pharmazeutische Zusammensetzung, die als Wirkstoff ein Produkt nach einem der Ansprüche 1 bis 8 enthält, das mit pharmakologisch verträglichen Verdünnungsmitteln oder Grundmassen kombiniert ist.

10. Verwendung eines Produkts nach einem der Ansprüche 1 bis 8 für die Herstellung eines Arzneimittels zur Behandlung von AIDS.

11. Verwendung nach Anspruch 10, **dadurch gekennzeichnet, dass** das verwendete Produkt 1-Ethoxymethyl-5-isopropyl-6-benzyluracil enthält.
